(19) Europäisches Patentamt
European Patent Office
Office européen des brevets

(11) Veröffentlichungsnummer: **0 205 022**
**B1**

(12) # EUROPÄISCHE PATENTSCHRIFT

(45) Veröffentlichungstag der Patentschrift: **10.01.90**

(51) Int. Cl.⁴: **C 07 D 403/04**, C 07 D 231/38, C 07 D 401/04, A 01 N 43/653, A 01 N 43/56, A 01 N 47/02

(21) Anmeldenummer: **86107122.3**

(22) Anmeldetag: **26.05.86**

(54) **1-Aryl-5-alkoximinoalkylamino-pyrazole.**

(30) Priorität: **07.06.85 DE 3520331**

(43) Veröffentlichungstag der Anmeldung: **17.12.86 Patentblatt 86/51**

(45) Bekanntmachung des Hinweises auf die Patenterteilung: **10.01.90 Patentblatt 90/2**

(84) Benannte Vertragsstaaten: **AT BE CH DE FR GB IT LI NL SE**

(56) Entgegenhaltungen:
**EP-A- 0 034 945**
**EP-A- 0 053 678**
**EP-A- 0 138 149**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Gehring, Reinhold, Dr., Dasnöckel 49, D-5600 Wuppertal 11 (DE)**
Erfinder: **Schallner, Otto, Dr., Noldeweg 22, D-4019 Monheim (DE)**
Erfinder: **Stetter, Jörg, Dr., Gellertweg 4, D-5600 Wuppertal 1 (DE)**
Erfinder: **Santel, Hans-Joachim, Dr., Grünstrasse 9a, D-5090 Leverkusen 1 (DE)**
Erfinder: **Schmidt, Robert R., Dr., Im Waldwinkel 110, D-5060 Bergisch-Galdbach 2 (DE)**
Erfinder: **Lürssen, Klaus, Dr., August-Kierspel-Strasse 151, D-5060 Bergisch-Gladbach 2 (DE)**

EP 0 205 022 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue 1-Aryl-5-alkoximinoalkylamino-pyrazole, mehrere Verfahren zu ihrer Herstellung und ihre Verwendung als Herbizide und Wachstumsregulatoren.

Es ist bereits bekannt, daß bestimmte 1-Aryl-5-amino-pyrazole, wie beispielsweise das 4-Cyano-5-propionamido-1-(2,3,4-trichlorphenyl)-pyrazol, herbizide Eigenschaften besitzen (vgl. z.B. DE-OS 3 226 513).

Die herbizide Wirksamkeit dieser vorbekannten Verbindungen gegenüber Unkräutern ist jedoch ebenso wie ihre Verträglichkeit gegenüber wichtigen Nutzpflanzen nicht immer in allen Anwendungsgebieten völlig zufriedenstellend.

Über eine Anwendung der vorbekannten Verbindungen als Wachstumsregulatoren ist nichts bekannt.

Es wurden neue 1-Aryl-5-alkoximinoalkylamino-pyrazole der allgemeinen Formel (I)

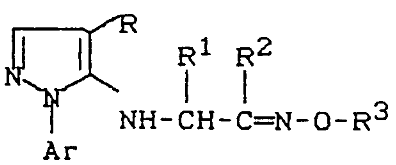

(I)

in welcher

R für Cyano, Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

$R^1$ für Wasserstoff oder für geradkettiges oder verzweigtes Alkyl mit 1 bis 8 Kohlenstoffatomen steht,

$R^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit 1 bis 4 Kohlenstoffatomen und 1 bis 9 gleichen oder verschiedenen Halogenatomen,

$R^3$ für Wasserstoff, für geradkettiges oder verzweigtes Alkyl mit 1 bis 8 Kohlenstoffatomen, für geradkettiges oder verzweigtes Alkenyl mit 3 bis 8 Kohlenstoffatomen, oder für jeweils gegebenenfalls einfach oder mehrfach, gleich oder verschieden durch Halogen, Cyano, Nitro, jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Benzyl oder Phenyl steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$, wobei

$R^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht,

gefunden.

Weiterhin wurde gefunden, daß man die neuen 1-Aryl-5-alkoximinoalkylamino-pyrazole der Formel (I)

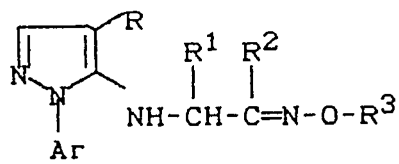

(I)

in welcher

R, $R^1$, $R^2$, $R^3$ und Ar die oben angegebenen Bedeutungen haben, erhält, wenn man

(a) 5-Amino-1-aryl-pyrazole der Formel (II)

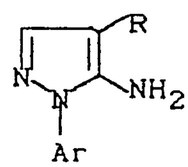

(II)

in welcher

R und Ar die oben angegebene Bedeutung haben, mit Alkoximinoalkylhalogeniden der Formel (III)

$$\text{Hal}-\underset{|}{\overset{R^1}{CH}}-\underset{|}{\overset{R^2}{C}}=N-OR^3 \qquad \text{(III)}$$

in welcher

$R^1$, $R^2$ und $R^3$ die oben angegebene Bedeutung haben und

Hal für Halogen steht,

gegebenenfalls in Gegenwart eines Verdünnungsmittels und gegebenenfalls in Gegenwart eines Säurebindemittels umsetzt, oder wenn man

(b) 5-Dialkoxyalkylamino-pyrazole der Formel (IV)

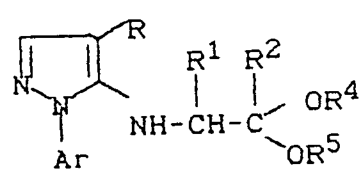

(IV)

in welcher

R, $R^1$, $R^2$ und Ar die oben angegebene Bedeutung haben und

$R^4$ und $R^5$ jeweils für geradkettiges oder verzweigtes Alkyl mit 1 bis 4 Kohlenstoffatomen stehen, mit Hydroxylamin-Derivaten der Formel (V)

$H_2N$-O-$R^3$ (V)

in welcher

$R^3$ die oben angegebene Bedeutung hat,

oder deren Säureadditionssalzen gegebenenfalls in Gegenwart eines Verdünnungsmittels und gegebenenfalls in Gegenwart eines Säurebindemittels umsetzt.

Schließlich wurde gefunden, daß die neuen 1-Aryl-5-alkoximinoalkylamino-pyrazole der Formel (I) herbizide, insbesondere auch selektiv-herbizide und pflanzenwachstumsregulatorische Eigenschaften besitzen.

Überraschenderweise zeigen die erfindungsgemäßen 1-Aryl-5-alkoximinoalkylamino-pyrazole der allgemeinen Formel (I) neben einer erheblich verbesserten herbiziden Wirksamkeit gegenüber Problemunkräutern gleichzeitig eine deutlich höhere Selektivität gegenüber wichtigen Kulturpflanzen als die aus dem Stand der Technik bekannten 1-Aryl-5-Amino-pyrazole, wie beispielsweise das 4-Cyano-5-proprionamido-1-(2,3,4-trichlorphenyl)-pyrazol, welche chemisch und wirkungsmässig naheliegende Verbindungen sind. Überraschenderweise zeigen die erfindungsgemäßen Verbindungen der Formel (I) auch eine gute Wirksamkeit als Wachstumsregulatoren.

Die erfindungsgemäßen 1-Aryl-5-alkoximinoalkylamino-pyrazole sind durch die Formel (I) allgemein definiert.

Bevorzugt sind Verbindungen der Formel (I), bei welchen

R für Cyano, Nitro oder für einen jeweils gegebenenfalls ein- bis dreifach, gleich oder verschieden durch Fluor, Chlor, Brom, Nitro, Methyl, Ethyl, n- und i-Propyl, Methoxy, Ethoxy, n- und i-Propoxy, Methylthio, Ethylthio, n- und i-Propylthio oder Trifluormethyl substituierten Heterocyclus der Formel

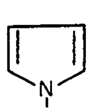 ; 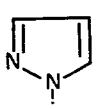 ; 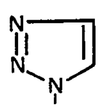 ; 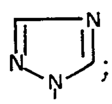 ; 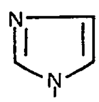 ; 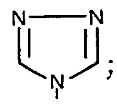 ; 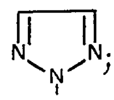 ; 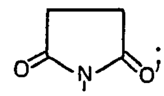 ; 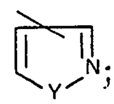 ;

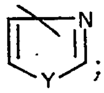 ; 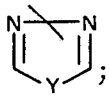 ; 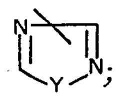 ; 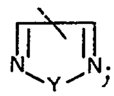 ; 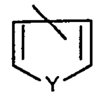 ; 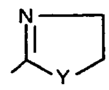 ; 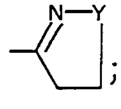 ; 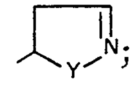 ; 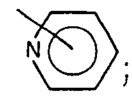 ;

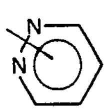 ; 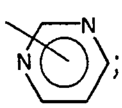 ; 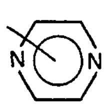 ; 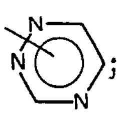 ; 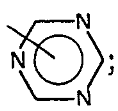 ; 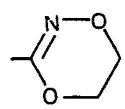 ; 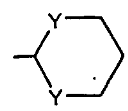 oder 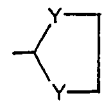

steht, wobei

Y jeweils für Sauerstoff, Schwefel oder einen N-Alkylrest mit 1 bis 4 Kohlenstoffatomen steht,

$R^1$ für Wasserstoff, Methyl, Ethyl, n- oder i-Propyl, n-, i-, s- oder t-Butyl steht,

$R^2$ für Wasserstoff, Methyl, Ethyl, n- oder i-Propyl, n-, i-,s- oder t-Butyl oder für gegebenenfalls ein- bis dreifach, gleich oder verschieden, durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy oder Trifluormethylthio substituiertes Phenyl steht,

$R^3$ für Wasserstoff, Methyl, Ethyl, n- oder i-Propyl, n-, i-, s- oder t-Butyl, Allyl, 2-Butenyl, 2-Methyl-2-butenyl oder für jeweils ein- bis dreifach, gleich oder verschieden substituiertes Benzyl oder Phenyl steht, wobei als Substituenten jeweils ausgewählt sind: Fluor, Chlor, Brom, Cyano, Nitro, Methyl, Ethyl, Methoxy, Ethoxy, Methylthio, Ethylthio oder Trifluormethyl und

Ar jeweils gegebenenfalls ein- bis fünffach, gleich oder verschieden substituiertes Phenyl, 2-Pyridyl oder 4-Pyridyl steht, wobei als Substituenten ausgewählt sind: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest -$S(O)_n$-$R^6$, wobei

$R^6$ für Amino, Methyl, Ethyl, Methylamino, Ethylamino, Dimethylamino, Diethylamino, Fluordichlormethyl, Difluormethyl, Tetrafluorethyl, Trifluorchlorethyl oder Trifluormethyl steht und

n für eine Zahl 0, 1 oder 2 steht.

Im einzelnen seien außer den bei den Herstellungsbeispielen genannten Verbindungen die folgenden 1-Aryl-5-alkoximinoalkylamino-pyrazole der allgemeinen Formel (I) genannt:

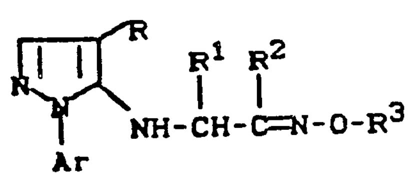

(I)

| R | $R^1$ | $R^2$ | $R^3$ | Ar |
|---|---|---|---|---|
| CN | $CH_3$ | H | $CH_3$ | 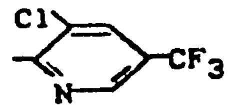 |
| $NO_2$ | $CH_3$ | H | $CH_3$ | 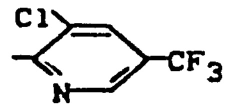 |
| CN | $CH_3$ | H | $CH_3$ | 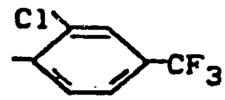 |
| $NO_2$ | $CH_3$ | H | $CH_3$ | 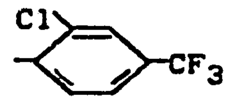 |
| CN | $CH_3$ | H | $CH_3$ | 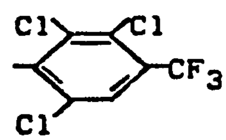 |
| $NO_2$ | $CH_3$ | H | $CH_3$ | 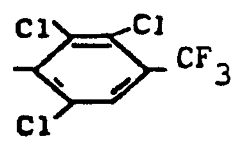 |
| CN | $CH_3$ | H | $CH_3$ | 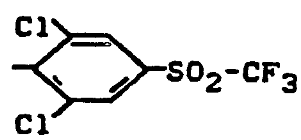 |
| CN | $CH_3$ | H | $CH_3$ | 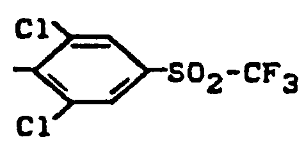 |
| 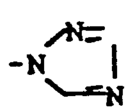 | $CH_3$ | H | $CH_3$ | 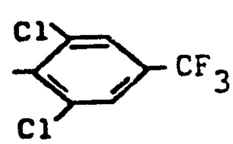 |

Verwendet man beispielsweise 5-Amino-4-cyano-1-(2,6-dichlor-4-trifluormethylphenyl)-pyrazol und 2-Brom-1-methoximino-propan als Ausgangsstoffe, so läßt sich der Reaktionsablauf des erfindungsgemäßen Verfahrens (a) durch das folgende Formelschema darstellen:

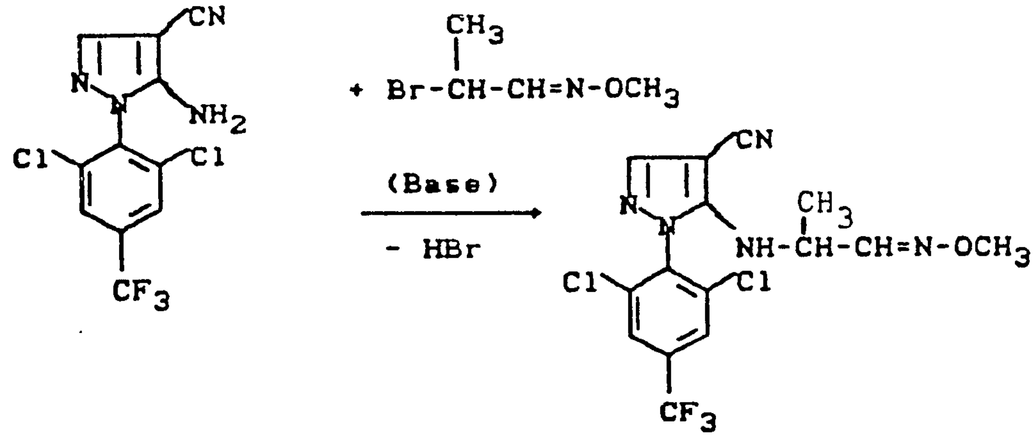


Verwendet man beispielsweise 4-Cyano-1-(3,5--dichlor-pyrid-2-yl)-5-(1,1-dimethoxyprop-2-ylamino)-pyrazol und O-Methylhydroxylamin-Hydrochlorid als Ausgangstoffe, so läßt sich der Reaktionsablauf des erfindungsgemäßen Verfahrens (b) durch das folgende Formelschema darstellen:

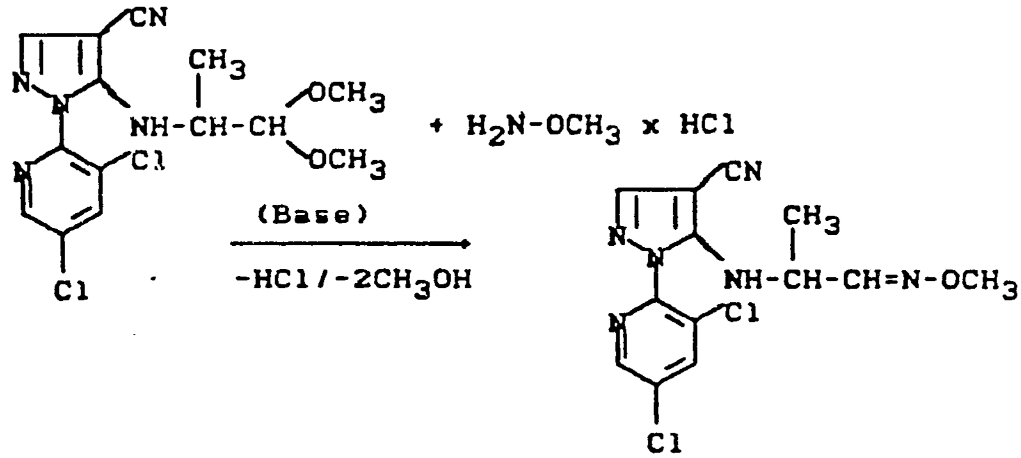


Die zur Durchführung des erfindungsgemäßen Verfahrens (a) als Ausgangsstoffe benötigten 5-Amino-1-aryl-pyrazole sind durch die Formel (II) allgemein definiert. In dieser Formel (II) stehen R und Ar vorzugsweise für diejenigen Reste, die bereits im Zusammenhang mit der Beschreibung der erfindungsgemäßen Stoffe der Formel (I) als bevorzugt für diese Substituenten genannt werden.

Die 5-Amino-1-aryl-pyrazole der Formel (II) sind teilweise bekannt (vgl. z.B. EP 26 034, EP 53 678, EP 34 945, DE-OS 3 226 496 oder DE-OS 3 408 727, DE-OS 3 402 308, DE-OS 3 423 101 und DE-OS 3 420 985).

Man erhält Verbindungen der Formel (II), in der R für Cyano oder für einen gegebenenfalls substituierten Heterocyclus steht, wobei der heterocyclische Rest bei der Beschreibung der Verbindung der Formel (I) definiert ist, wenn man Arylhydrazine der Formel (VI),

$$Ar\text{-}NH\text{-}NH_2 \qquad (VI)$$

in welcher

Ar die oben angegebene Bedeutung hat, mit Acrylnitril-Derivaten der Formel (VIIa)

$$Z\text{-}CH=C\genfrac{}{}{0pt}{}{CN}{R'} \qquad (VIIa)$$

in welcher

R' mit Ausnahme von Nitro die bei der Beschreibung der Verbindungen der Formel (I) für R angegebenen Bedeutungen hat und

Z für Halogen, Hydroxy, Alkoxy oder Dialkylamino steht,

zunächst in einer 1. Stufe gegebenenfalls in Gegenwart eines Verdünnungsmittels, wie beispielsweise Eisessig oder Ethanol, und gegebenenfalls in Gegenwart eines Reaktionshilfsmittels, wie beispielsweise Natriumacetat, bei Temperaturen zwischen −20 und +20°C umsetzt zu den Arylhydrazin-Derivaten der Formel (VIII),

$$AR\text{-}NH\text{-}NH\text{-}CH=C\genfrac{}{}{0pt}{}{CN}{R'} \qquad (VIII)$$

in welcher

Ar und R' die oben angegebene Bedeutung haben, und die Zwischenprodukte der Formel (VIII) in einer 2. Stufe gegebenenfalls in Gegenwart eines Verdünnungsmittels, wie beispielsweise Ethylenglykolmonoethylether, und gegebenenfalls in Gegenwart eines sauren Katalysators, wie beispielsweise Schwefel- oder Phosphorsäure bei Temperaturen zwischen +50 und +150°C cyclisiert.

Die Reaktion kann auch direkt in einem Reaktionsschritt ohne Isolierung der Zwischenprodukte der Formel (VIII) gegebenenfalls in Gegenwart eines Verdünnungsmittels, wie beispielsweise Ethylenglykolmonoethylether oder Ethanol, bei Temperaturen zwischen +50 und +150°C durchgeführt werden.

Man erhält Verbindungen der Formel (II) in der R für Nitro steht, wenn man Arylhydrazine der Formel (VI),

Ar-NH-$NH_2$ (VI)

in welcher

Ar die oben angegebene Bedeutung hat,

mit 2-Halogenacrylnitrilen der Formel (VIIb)

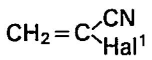 (VIIb)

in welcher

Hal für Halogen, insbesondere für Chlor oder Brom steht,

nach der oben beschriebenen Verfahrensweise entweder in einer Zweistufen- oder Einstufenreaktion umsetzt zu den in 4-Position unsubstituierten 5-Amino-pyrazolen der Formel (IX),

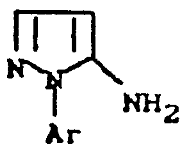 (IX)

in welcher

Ar die oben angegebene Bedeutung hat, und diese in einer Folgereaktion mit einem Nitrierungsmittel, wie beispielsweise Salpetersäure, gegebenenfalls in Gegenwart eines Verdünnungsmittels, wie beispielsweise Eisessig, und gegebenenfalls in Gegenwart eines Reaktionshilfsmittels, wie beispielsweise Acetanhydrid, bei Temperaturen zwischen −20 und +50°C in 4-Position des Parazol-Ringes nitriert.

Dabei kann es gegebenenfalls von Vorteil sein, die Amino-Gruppe in der 5-Position des Pyrazol-Ringes vor der Nitrierungsreaktion mit Hilfe einer Schutzgruppe, beispielsweise durch Acylierung, zu schützen und die Schutzgruppe nach erfolgter Nitrierung, beispielsweise durch Verseifung mit wäßriger oder alkoholischer Base, wieder abzuspalten.

Die zur Durchführung des erfindungsgemäßen Verfahrens (a) weiterhin als Ausgangsstoffe benötigte Alkoximinoalkylhalogenide sind durch die Formel (III) allgemein definiert. In dieser Formel (III) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Alkoximinoalkylhalogenide der Formel (III) sind bekannt (vgl. z.B. DE-OS 2 922 759 oder US-PS 4 337 268) oder lassen sich nach bekannten Verfahren in einfacher analoger Weise herstellen.

Die Arylhydrazine der Formel (VI) sind ebenfalls bekannt [vgl. z.B. US-PS 4 127 575; US-PS 3 609 158; DE-OS 2 558 339; J. chem. Soc. C, 167-174 (1971)] oder können nach bekannten Verfahren in einfacher analoger Weise erhalten werden [vgl. z.B. Houben-Weyl «Methoden der organischen Chemie» Band X/2 S. 203, Thieme Verlag Stuttgart, (1967)], indem man beispielsweise die Verbindungen der Formel (X),

Ar-$NH_2$ (X)

in welcher

Ar die oben angegebene Bedeutung hat,

mit Natriumnitrit in Gegenwart einer Säure, wie beispielsweise Schwefelsäure, und dann mit Zinn-IIchlorid ebenfalls in Gegenwart einer Säure, wie beispielsweise Salzsäure, bei Temperaturen zwischen −20 und +80°C umsetzt,

oder wenn man Verbindungen der Formel (XI),

Ar-$Hal^2$ (XI)

in welcher

Ar die oben angegebene Bedeutung hat und

$Hal^2$ für Halogen, insbesondere für Fluor, Chlor oder Brom steht,

mit Hydrazinhydrat gegebenenfalls in Gegenwart eines Verdünnungsmittels, wie beispielsweise Pyridin oder Dioxan, bei Temperaturen zwischen 0 und +150°C umsetzt.

Die Acrylnitril-Derivate der Formel (VIIa), die 2-Halogenacrylnitrile der Formel (VIIb), die Verbindungen der Formel (X) und Verbindungen der Formel (XI) sind allgemein bekannte Verbindungen der organischen Chemie.

Die zur Durchführung des erfindungsgemäßen Verfahrens (b) als Ausgangsstoffe benötigten 5-Dialkoxyalkylamino-pyrazole sind durch die Formel (IV) allgemein definiert. In dieser Formel (IV) stehen R, $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt werden.

$R^4$ und $R^5$ stehen vorzugsweise für Methyl oder Ethyl.

Die 5-Dialkoxyalkylamino-pyrazole der Formel (IV), in der R für Cyano steht, sind Gegenstand der eigenen Patentanmeldung D-OS 3 426 424.

Die 5-Dialkoxyalkylamino-pyrazole der Formel (IV), in der R für Nitro oder für einen gegebenenfalls substituierten Heterocyclus steht, sind neu und sind durch die Formel (IVa)

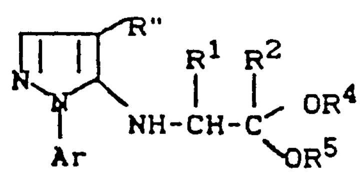 (IVa)

allgemein definiert.

In der Formel (IVa) stehen

R für Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

$R^1$ für Wasserstoff oder für geradkettiges oder verzweigtes Alkyl mit 1 bis 8 Kohlenstoffatomen steht,

$R^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^4$ und $R^5$ jeweils unabhängig für geradkettiges oder verzweigtes Alkyl mit 1 bis 4 Kohlenstoffatomen stehen und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$, wobei

$R^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht.

In der Formel (IVa) stehen R'' vorzugsweise für diejenige Bedeutung von R, mit Ausnahme von Cyano, welche bereits im Zusammenhang mit der Beschreibung der erfindungsgemäßen Stoffe der Formel (I) als bevorzugt für diesen Substituenten genannt wurde, $R^1$, $R^2$ und Ar für diejenigen Bedeutungen, die bereits im Zusammenhang mit der Beschreibung der erfindungsgemäßen Stoffe der Formel (I) als bevorzugt für $R^1$, $R^2$ und Ar genannt wurden. $R^4$ und $R^5$ stehen vorzugsweise für Methyl oder Ethyl.

Man erhält Verbindungen der Formel (IVa), wenn man 5-Amino-1-aryl-pyrazole der Formel (IIa),

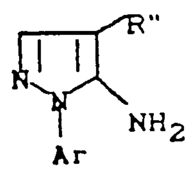

(IIa)

in welcher

R'' und Ar die oben angegebene Bedeutung haben,

mit Nitritverbindungen der Formel (XI),

$R^7\text{-O-N=O}$ (XII)

in welcher

$R^7$ für Wasserstoff, ein Alkalimetallkation oder Alkyl steht,

in Gegenwart einer Halogenwasserstoffsäure oder in Gegenwart eines Haloforms und einer Katalysatorsäure, wie beispielsweise Schwefelsäure, bei Temperaturen zwischen −20 und +80°C diazotiert und die so erhältlichen 5-Halogenpyrazole der Formel (XIII),

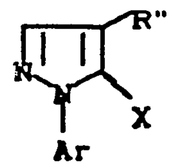

(XIII)

in welcher

R'' und Ar die oben angegebene Bedeutung haben und

X für Halogen, insbesondere für Chlor oder Brom steht, mit Dialkoxyalkylaminen der Formel (XIV),

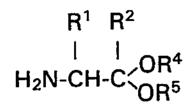

(XIV)

in welcher

$R^1$, $R^2$, $R^4$ und $R^5$ die oben angegebene Bedeutung haben,

bei Temperaturen zwischen +50 und +250°C umsetzt.

Die Diazotierung der Verbindungen der Formel (IIa) wird in üblicher Art und Weise durchgeführt [vgl. «Organikum» 15. Auflage, VEB Deutscher Verlag der Wissenschaften, Berlin 1981, S. 652 ff; J. chem. Soc. C 1249 (1966), Rev. Latinoam. Quim. 13, 100-102 (1982)].

Nach dem oben beschriebenen Verfahren lassen sich alle Verbindungen der Formel (IV) herstellen.

Die 5-Amino-1-aryl-pyrazole der Formeld (IIa) sind teilweise bekannt (vgl. z.B. EP 26 034, EP 53 678 oder DE-OS 3 408 727, DE-OS 3 402 308, DE-OS 3 423 101 und DE-OS 3 420 985).

In der Formel (IIa) stehen R'' und Ar bevorzugt für diejenigen Bedeutungen von R (ausgenommen Cyano) und Ar, welche bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (IIa) sind erhältlich nach den gleichen Verfahren, die zur Herstellung der 5-Amino-1-aryl-pyrazole der Formel (II) beschrieben wurden.

Die Nitritverbindungen der Formel (XII) und die Dialkoxyalkylamine der Formel (XIV) sind allgemein bekannte Verbindungen der organischen Chemie. Die als Zwischenprodukte erhältlichen 5-Halogenpyrazole der Formel (XIII) sind Gegenstand eigener Patentanmeldung D-OS 3 501 323 und alternativ auch nach den dort beschriebenen Herstellungsmethoden erhältlich.

Die zur Durchführung des erfindungsgemäßen

Verfahrens (b) weiterhin als Ausgangsstoffe benötigten Hydroxylamin-Derivate sind durch die Formel (V) allgemein definiert. In dieser Formel (V) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Hydroxylamin-Derivate der Formel (V) und deren Säureadditionssalze sind allgemein bekannte Verbindungen der organischen Chemie.

Als Verdünnungsmittel zur Durchführung des erfindungsgemäßen Verfahrens (a) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Dimethylformamid, Dimethylacetamid, N-Methylformamilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 und +150°C, vorzugsweise bei Temperaturen zwischen +20 und +120°C.

Zur Durchführung des erfindungsgemäßen Verfahrens (a) setzt man pro Mol an 5-Amino-1-aryl-pyrazol der Formel (II) im allgemeinen 1,0 bis 10,0 Mol, vorzugsweise 1,0 bis 5,0 Mol an Alkoximinoalkylhalogenid der Formel (III) und gegebenenfalls 1,0 bis 5,0 Mol, vorzugsweise 1,0 bis 2,5 Mol, an Säurebindemittel ein.

Die Reaktionsdurchführung, Aufarbeitung und Isolierung der Reaktionsprodukte der Formel (I) erfolgt nach üblichen Verfahren.

Als Verdünnungsmittel zur Durchführung des erfindungsgemäßen Verfahrens (b) kommen ebenfalls inerte organische Lösungsmittel in Frage.

Vorzugsweise verwendet man die bei Verfahren (a) aufgezählten Lösungsmittel oder Alkohole, wie Methanol oder Ethanol.

Die Reaktionstemperaturen können bei der Durchführung des erfindungsgemäßen Verfahrens (b) in einem größeren Bereich variiert werden. Im allgemeinen arbeitet man bei Temperaturen zwischen 0 und +150°C, vorzugsweise bei Temperaturen zwischen +20 und +100°C.

Zur Durchführung des erfindungsgemäßen Verfahrens (b) setzt man pro Mol an 5-Dialkoxyalkyl-amino-pyrazol der Formel (IV) im allgemeinen 1,0 bis 3,0 Mol, vorzugsweise 1,0 bis 1,5 Mol an Hydroxylamin-Derivat der Formel (V) und gegebenenfalls 1,0 bis 3,0 Mol, vorzugsweise 1,0 bis 1,5 Mol, an Säurebindemittel ein.

Die Reaktionsdurchführung, Aufarbeitung und Isolierung der Reaktionsprodukte der Formel (I) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycine, Beta, Daucus, Phaseolus, Pisum, Solanum, Linum, Ipomoea,Vicia, Nicotiana, Lycopersicon, Arachis, Brassica, Lactuca, Cucumis, Cucurbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Penicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur

selektiven Bekämpfung mono- und dikotyler Unkräuter in mono- und dikotylen Kulturen, wie beispielsweise Weizen und Baumwolle einsetzen.

Auch die Vorprodukte der Formel (IV) zeigen eine gute herbizide Wirksamkeit.

Die erfindungsgemäßen Wirkstoffe greifen auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können z.B. zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des

Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfrost zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Darüber hinaus besitzen die erfindungsgemäßen Wirkstoffe der Formel (I) auch eine insektizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen als solche oder in Mischung mit anderen bekannten Wirkstoffen vorliegen, wie Fungizide, Insektizide, Nematizide, Akarizide und anderen Herbiziden, sowie in Mischungen mit Düngemitteln, Schutzstoffen gegen Vogelfraß, Bodenstrukturverbesserungmitteln und anderen Wachstumsregulatoren, wobei Fertigformulierungen oder Tankmischungen möglich sind.

Für die Mischungen mit anderen Herbiziden kommen bekannte Herbizide wie z.B. 1-Amino-6-ethylthio-3-(2,2-dimethylpropyl)-1,3,5-triazin-2,4(1H, 3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5-(4H)-on zur Unkrautbekämpfung in Zuckerrüben und 4-Amino-6-(1,1-dimethylethyl-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; Methyl-5-(2,4-dichlorphenoxy)-2-nitrobenzoat; 3,5-Diiod-4-hydroxybenzonitril; 3-Isopropyl-

-2,1,3-benzothiadiazin-4-on-2,2-dioxid; 2-Chlor-N--[[(4-methoxy-6-methyl-1,3,5-triazin-2-yl)-amino]--carbonyl]-benzolsulfonamid; 4-Ethylamino-2-t-butylamino-6-methylthio-S-triazin; N-(1-Ethylpropyl)--3,4-dimethyl-2,6-dinitroanilin; 6-Chlor-3-phenyl--pyridazin-4-yl-S-octyl-thiocarbonat; N,N-Diisopropyl-S-(2,3,3-trichlorallyl)-thiocarbamat; N-Methyl-2-(1,3-benzthiazol-2-yloxy)-acetanilid; N,N-Di--n-propyl-thiocarbamidsäure-S-ethylester; exo-1--Methyl-4-(1-methylethyl)-2-(2-methylphenylmethoxy)-7-oxabicyclo-[2.2.1]-heptan; 2-[4-[[3-Chlor-5--(trifluormethyl)-2-pyridinyl]-oxy]-phenoxy]-propansäure bzw. -propansäureethylester; [(4-Amino-3,5--dichlor-6-fluor-2-pyridinyl)-oxy]-essigsäure bzw. -1-methylheptylester; 3,5-Dibrom-4-hydroxy-benzonitril; 2-[4-(2,4-Dichlorphenoxy)-phenoxy]-propionsäuremethylester; 4,6-Dinitro-2-(1-methylpropyl)-phenol; 2,6-Dinitro-4-trifluormethyl-N,N-dipropylanilin; 2-[4-(3,5-Dichlorpyrid-2-yloxy)-phenoxy]--propionsäure-(2-benzyloxyethylester), -(trimethylsilylmethylester) oder -(2,2-diethoxyethylester); sind gegebenenfalls von Vorteil.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren richtet sich der Anwendungszeitraum nach den klimatischen und vegetativen Gegebenheiten.

Die Herstellung und Verwendung der erfindungsgemäßen Wirkstoffe geht aus den nachfolgenden Beispielen hervor.

*Herstellungsbeispiele*

*Beispiel 1*

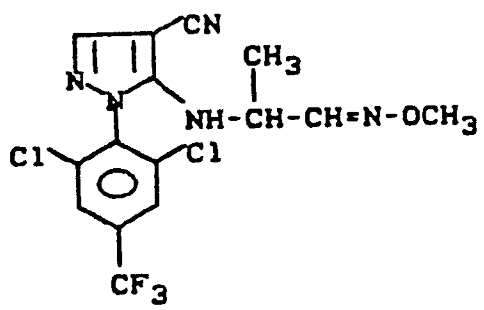

(Verfahren a)

10,5 g (0,033 mol) 5-Amino-4-cyano-1-(2,6-dichlor-4-trifluor-methylphenyl)-pyrazol, 9,1 g (0,066 mol) Kaliumcarbonat und 12,7 g (0,073 mol) 2-Brom-1-methoximinopropan in 100 ml Acetonitril werden unter Rückfluß so lange erhitzt, bis im Dünnschichtchromatogramm kein Ausgangsprodukt mehr nachweisbar ist (Dauer ca. 2 Tage). Die abgekühlte Reaktionsmischung wird filtriert, flüchtige Bestandteile im Vakuum bei 70°C Badtemperatur abdestilliert und der ölige Rückstand mit Petrolether zur Kristallisation gebracht.

Man erhält 11,1 g (83% der Theorie) an 4-Cyano--5-(1-methoximinoprop-2-ylamino)-1(2,6-dichlor-4--trifluor-methylphenyl)-pyrazol vom Schmelzpunkt 117°C-119°C.

In entsprechender Weise und gemäß den allgemeinen Angaben zur Herstellung erhält man die folgenden 1-Aryl-5-alkoximino-alkylamino-pyrazole der allgemeinen Formel (I):

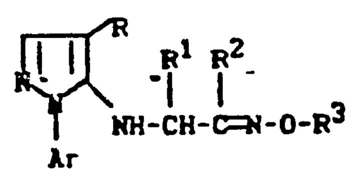

(I)

TABELLE 1

| Beispiel Nr. | R | $R^1$ | $R^2$ | $R^3$ | Ar | physikalische Eigenschaften |
|---|---|---|---|---|---|---|
| 2 | CN | $CH_3$ | H | $CH_3$ | 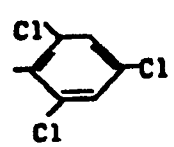 | Fp. 121°C |
| 3 | $NO_2$ | $CH_3$ | H | $CH_3$ | 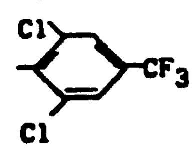 | Fp. 81°C |

TABELLE 1 (Fortsetzung)

| Beispiel Nr. | R | $R^1$ | $R^2$ | $R^3$ | Ar | physikalische Eigenschaften |
|---|---|---|---|---|---|---|
| 4 | CN | $CH_3$ | H | $CH_3$ | 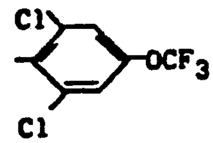 | Fp. 112-115°C |
| 5 | CN | H | H | $CH_3$ | 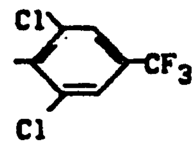 | $^1$H-NMR 3,7 ppm* |
| 6 | CN | $CH_3$ | H | $CH_3$ | 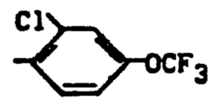 | $^1$H-NMR 3,7 ppm* |
| 7 | CN | $CH_3$ | H | $CH_3$ | 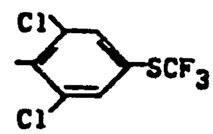 | $^1$H-NMR 3,7 ppm* |
| 8 | CN | $CH_3$ | H | $CH_3$ | 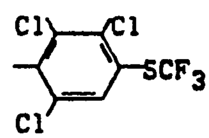 | $^1$H-NMR 3,7 ppm* |
| 9 | CN | $CH_3$ | H | $CH_3$ | 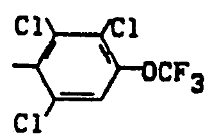 | $^1$H-NMR 3,7 ppm* |
| 10 | $NO_2$ | $CH_3$ | H | $CH_3$ | 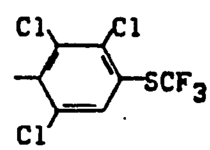 | $^1$H-NMR 3,7 ppm* |
| 11 | $NO_2$ | $CH_3$ | H | $CH_3$ | 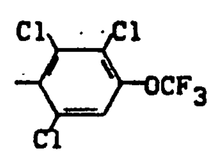 | Fp. 83°C |
| 12 | CN | $CH_3$ | H | $CH_3$ | 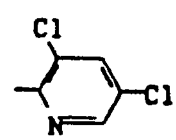 | Fp. 110-120°C |

* Die $^1$H-NMR Spektren wurden in Chloroform-$d_3$ mit Tetramethylsilan als innerem Standard aufgenommen. Angegeben ist die Chemische Verschiebung der Methoximinogruppe als δ-Wert in ppm.

*Anwendungsbeispiele*

In den Anwendungsbeispielen wurde die nachstehend aufgeführte Verbindung als Vergleichssubstanz eingesetzt:

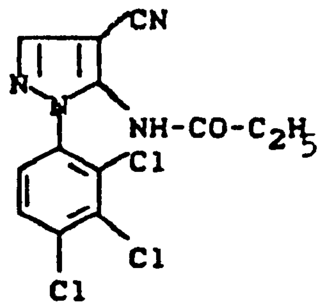

(A)

4-Cyano-5-propionamido-1-(2,3,4-trichlorphenyl)-pyrazol (bekannt aus DE-OS 3 226 513).

*Beispiel A*

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)
100% = totale Vernichtung

Eine deutliche Überlegenheit in der Wirksamkeit ebenso wie in der Nutzpflanzenselektivität gegenüber der Vergleichssubstanz (A) zeigt in diesem Test z.B. die Verbindung gemäß dem Herstellungsbeispiel 1.

*Beispiel B*

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)
100% = totale Vernichtung

Eine deutliche Überlegenheit in der Wirksamkeit sowie in der Nutzpflanzenselektivität gegenüber der Vergleichssubstanz (A) zeigt in diesem Test z.B. die Verbindung gemäß dem Herstellungsbeispiel 1.

*Beispiel C*

*Entlaubung und Austrocknung der Blätter bei Baumwolle*

Lösungsmittel: 30 Gewichtsteile Dimethylformamid
Emulgator: 1 Gewichtsteil Polyoxyethylen-Sorbitanmonolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

Eine deutliche Überlegenheit im Vergleich zur unbehandelten Kontrolle zeigen in diesem Test beispielsweise die Verbindungen gemäß den Herstellungsbeispielen 1 und 2.

**Patentansprüche**

1. 1-Aryl-5-alkoximinoalkylamino-pyrazole der Formel (I)

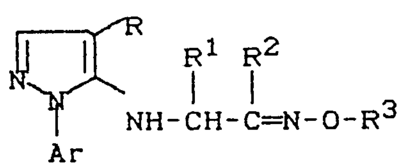

(I)

in welcher

R für Cyano, Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

$R^1$ für Wasserstoff oder für geradkettiges oder verzweigtes Alkyl mit 1 bis 8 Kohlenstoffatomen steht,

$R^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit 1 bis 4 Kohlenstoffatomen und 1 bis 9 gleichen oder verschiedenen Halogenatomen,

$R^3$ für Wasserstoff, für geradkettiges oder verzweigtes Alkyl mit 1 bis 8 Kohlenstoffatomen, für geradkettiges oder verzweigtes Alkenyl mit 3 bis 8 Kohlenstoffatomen, oder für jeweils gegebenenfalls einfach oder mehrfach, gleich oder verschieden durch Halogen, Cyano, Nitro, jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Benzyl oder Phenyl steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$, wobei

$R^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht.

2. 1-Aryl-5-alkoximinoalkylamino-pyrazole der Formel (I) gemäß Anspruch 1, in welcher

R für Cyano, Nitro oder für einen gegebenenfalls ein- bis dreifach, gleich oder verschieden durch Fluor, Chlor, Brom, Nitro, Methyl, Ethyl, n- und i-Propyl, Methoxy, Ethoxy, n- und i-Propoxy, Methylthio, Ethylthio, n- und i-Propylthio oder Trifluormethyl substituierten Heterocyclus der Formel

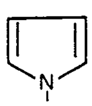; 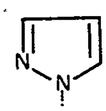; 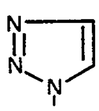; 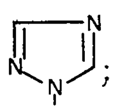; 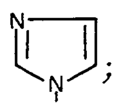; 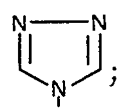; 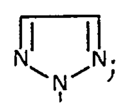; 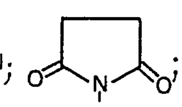; 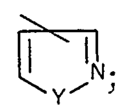;

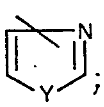; 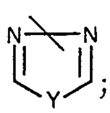; 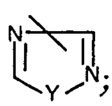; 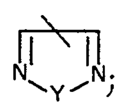; 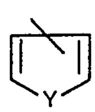; 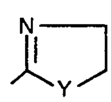; 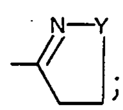; 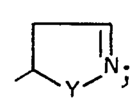; 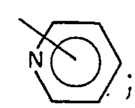;

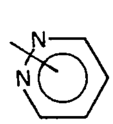; 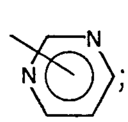; 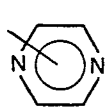; 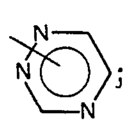; 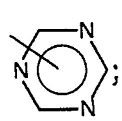; 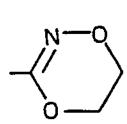; 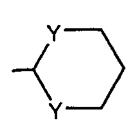 oder 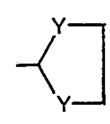

steht, wobei

Y jeweils für Sauerstoff, Schwefel oder einen N-Alkylrest mit 1 bis 4 Kohlenstoffatomen steht,

$R^1$ für Wasserstoff, Methyl, Ethyl, n- oder i-Propyl, n-, i-, s- oder t-Butyl steht,

$R^2$ für Wasserstoff, Methyl, Ethyl, n- oder i-Propyl, n-, i-, s- oder t-Butyl oder für gegebenenfalls einbis dreifach, gleich oder verschieden, durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy oder Trifluormethylthio substituiertes Phenyl steht,

$R^3$ für Wasserstoff, Methyl, Ethyl, n- oder i-Propyl, n-, i-, s- oder t-Butyl, Allyl, 2-Butenyl, 2-Methyl-2-butenyl oder für jeweils ein- bis dreifach, gleich oder verschieden substituiertes Benzyl oder Phenyl steht, wobei als Substituenten jeweils ausgewählt sind: Fluor, Chlor, Brom, Cyano, Nitro, Methyl,

Ethyl, Methoxy, Ethoxy, Methylthio, Ethylthio oder Trifluormethyl und

Ar jeweils gegebenenfalls ein- bis fünffach, gleich oder verschieden substituiertes Phenyl, 2-Pyridyl oder 4-Pyridyl steht, wobei als Substituenten ausgewählt sind: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest -S(O)$_n$-R$^6$, wobei

R$^6$ für Amino, Methyl, Ethyl, Methylamino, Ethylamino, Dimethylamino, Diethylamino, Fluordichlormethyl, Difluormethyl, Tetrafluorethyl, Trifluorchlorethyl oder Trifluormethyl steht und

n für eine Zahl 0, 1 oder 2 steht.

3. Verfahren zur Herstellung von 1-Aryl-5-alkoximinoalkylamino-pyrazolen der Formel (I)

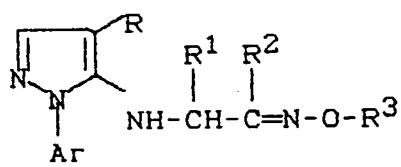 (I)

in welcher

R für Cyano, Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

R$^1$ für Wasserstoff oder für geradkettiges oder verzweigtes Alkyl mit 1 bis 8 Kohlenstoffatomen steht,

R$^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit 1 bis 4 Kohlenstoffatomen und 1 bis 9 gleichen oder verschiedenen Halogenatomen,

R$^3$ für Wasserstoff, für geradkettiges oder verzweigtes Alkyl mit 1 bis 8 Kohlenstoffatomen, für geradkettiges oder verzweigtes Alkenyl mit 3 bis 8 Kohlenstoffatomen, oder für jeweils gegebenenfalls einfach oder mehrfach, gleich oder verschieden durch Halogen, Cyano, Nitro, jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Benzyl oder Phenyl steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest -S(O)$_n$-R$^6$, wobei

R$^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht,

dadurch gekennzeichnet, daß man

(a) 5-Amino-1-aryl-pyrazole der Formel (II)

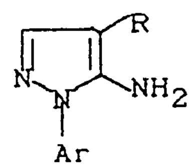 (II)

in welcher

R und Ar die oben angegebene Bedeutung haben, mit Alkoximinoalkylhalogeniden der Formel (III)

$$\mathrm{Hal{-}\overset{R^1}{\overset{|}{C}H}{-}\overset{R^2}{\overset{|}{C}}{=}N{-}OR^3} \qquad \text{(III)}$$

in welcher

R$^1$, R$^2$ und R$^3$ die oben angegebene Bedeutung haben und

Hal für Halogen steht,

gegebenenfalls in Gegenwart eines Verdünnungsmittels und gegebenenfalls in Gegenwart eines Säurebindemittels umsetzt, oder daß man

(b) 5-Dialkoxyalkylamino-pyrazole der Formel (IV)

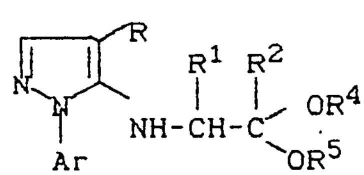 (IV)

in welcher

R, R$^1$, R$^2$ und Ar die oben angegebene Bedeutung haben und

$R^4$ und $R^5$ jeweils für geradkettiges oder verzweigtes Alkyl mit 1 bis 4 Kohlenstoffatomen stehen, mit Hydroxylamin-Derivaten der Formel (V)

$H_2N$-O-$R^3$ (V)

in welcher

$R^3$ die oben angegebene Bedeutung hat, oder deren Säureadditionssalzen gegebenenfalls in Gegenwart eines Verdünnungsmitteln und gegebenenfalls in Gegenwart eines Säurebindemittels umsetzt.

4. Herbizide und pflanzenwuchsregulierende Mittel, gekennzeichnet durch einen Gehalt an mindestens einem 1-Aryl-5-alkoximinoalkylamino-pyrazol der Formel (I) gemäß den Ansprüchen 1 bis 3.

5. Verfahren zur Bekämpfung von Unkräutern, dadurch gekennzeichnet, daß man 1-Aryl-5-alkoximinoalkylamino-pyrazole der Formel (I) gemäß den Ansprüchen 1 bis 3 auf die Unkräuter und/oder ihren Lebensraum einwirken läßt.

6. Verwendung von 1-Aryl-5-alkoximinoalkylamino-pyrazolen der Formel (I) gemäß den Ansprüchen 1 bis 3, zur Bekämpfung von Unkräutern und/oder als Pflanzenwachstumsregulatoren.

7. Verfahren zur Herstellung von herbiziden und/oder pflanzenwuchsregulierenden Mitteln, dadurch gekennzeichnet, daß man 1-Aryl-5-alkoximinoalkylamino-pyrazole der Formel (I) gemäß den Ansprüchen 1 bis 3 mit Streckmitteln und/oder oberflächenaktiven Substanzen vermischt.

8. Dialkoxyalkylamino-pyrazole der Formel (IVa),

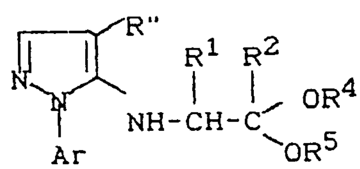

(IVa)

in welcher

R'' für Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

$R^1$ für Wasserstoff oder für geradkettiges oder verzweigtes Alkyl mit 1 bis 8 Kohlenstoffatomen steht,

$R^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

$R^4$ für geradkettiges oder verzweigtes Alkyl mit 1 bis 4 Kohlenstoffatomen steht,

$R^5$ für geradkettiges oder verzweigtes Alkyl mit 1 bis 4 Kohlenstoffatomen steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest -$S(O)_n$-$R^6$, wobei

$R^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht.

9. Verfahren zur Herstellung von 5-Dialkoxyalkylamino-pyrazolen der Formel (IVa)

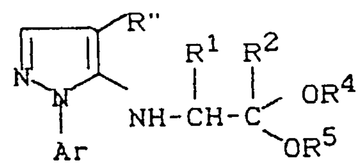

(IVa)

in welcher

R'' für Nitro oder für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschiedene Heteroatome aus der Gruppe Stickstoff, Sauerstoff und Schwefel enthält und der über ein Kohlenstoff- oder ein Stickstoffatom verknüpft sein kann, wobei als Substituenten ausgewählt sind: Halogen, Nitro, sowie jeweils geradkettiges oder verzweigtes Alkyl, Alkoxy, Alkylthio mit jeweils 1 bis 4 Kohlenstoffatomen oder Halogenalkyl mit 1 bis 4 Kohlenstoffatomen und 1 bis 9 gleichen oder verschiedenen Halogenatomen,

$R^1$ für Wasserstoff oder für geradkettiges oder verzweigtes Alkyl mit 1 bis 8 Kohlenstoffatomen steht,

$R^2$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten ausgewählt sind: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den einzelnen Alkylteilen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen

$R^4$ für geradkettiges oder verzweigtes Alkyl mit 1 bis 4 Kohlenstoffatomen steht,

$R^5$ für geradkettiges oder verzweigtes Alkyl mit 1 bis 4 Kohlenstoffatomen steht und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten ausgewählt sind: Cyano, Nitro, Halogen, jeweils geradkettiges oder verzweigtes Alkyl, Alkoxy oder Alkoxycarbonyl mit 1 bis 4 Kohlenstoffatomen in den einzelnen Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$, wobei

$R^6$ für Amino, für jeweils geradkettiges oder verzweigtes Alkyl, Alkylamino, Dialkylamino mit jeweils 1 bis 4 Kohlenstoffatomen in den einzelnen Alkylteilen oder Halogenalkyl mit 1 bis 4 Kohlenstoffatomen und mit 1 bis 9 gleichen oder verschiedenen Halogenatomen steht und

n für eine Zahl 0, 1 oder 2 steht,

dadurch gekennzeichnet, daß man 5-Amino-1-aryl-pyrazole der Formel (IIa)

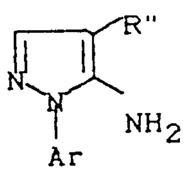

(IIa)

in welcher

R'' und Ar die oben angegebene Bedeutung haben,

mit Nitritverbindungen der Formel (XII)

$R^7-O-N=O$ (XII)

in welcher

$R^7$ für Wasserstoff, ein Alkalimetallkation oder Alkyl steht,

in Gegenwart einer Halogenwasserstoffsäure oder in Gegenwart eines Haloforms und einer Katalysatorsäure, bei Temperaturen zwischen −20 °C und 80 °C diazotiert und die so erhältlichen 5-Halogenpyrazole der Formel (XIII)

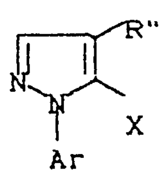

(XIII)

in welcher

R'' und Ar die oben angegebene Bedeutung haben und

X für Halogen, insbesondere für Chlor oder Brom steht,

mit Dialkoxyalkylaminen der Formel (XIV)

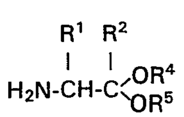

(XIV)

in welcher

$R^1$, $R^2$, $R^4$ und $R^5$ die oben angegebene Bedeutung haben,

bei Temperaturen zwischen +50 °C und +250 °C umsetzt.

**Claims**

1. 1-Aryl-5-alkoximinoalkylamino-pyrazoles of the formula (I)

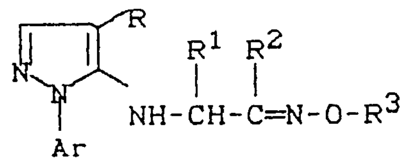

(I)

in which

R represents cyano or nitro, or represents a saturated or unsaturated, five-membered or six-membered heterocyclic radical which is optionally monosubstituted or polysubstituted by identical or different substituents, contains one to three identical or different hetero atoms from the group comprising nitrogen, oxygen and sulphur and can be linked via a carbon or a nitrogen atom, the substituents chosen being: halogen, nitro and in each case straight-chain or branched alkyl, alkoxy and alkylthio with in each case 1 to 4 carbon atoms or halogenoalkyl with 1 to 4 carbon atoms and 1 to 9 identical or different halogen atoms,

$R^1$ represents hydrogen, or represents straight-chain or branched alkyl with 1 to 8 carbon atoms,

$R_2$ represents hydrogen, or represents straight-chain or branched alkyl with 1 to 8 carbon atoms, or represents phenyl which is optionally monosubstituted or polysubstituted by identical or different substituents, the substituents chosen being: halogen, cyano, nitro, in each case straight-chain or branched alkyl, alkoxy, alkylthio or alkoxycarbonyl with in each case 1 to 4 carbon atoms in the individual alkyl parts or in each case straight-chain or branched halogenoalkyl, halogenoalkoxy and halogenoalkylthio with in each case 1 to 4 carbon atoms and 1 to 9 identical or different halogen atoms,

$R^3$ represents hydrogen, or represents straight-chain or branched alkyl with 1 to 8 carbon atoms, or represents straight-chain or branched alkenyl with 3 to 8 carbon atoms, or represents benzyl or phenyl, in each case optionally monosubstituted or polysubstituted by identical or different substituents from the group comprising halogen, cyano, nitro and in each case straight-chain or branched alkyl, alkoxy and alkylthio with in each case 1 to 4 carbon atoms and halogenoalkyl with 1 to 4 carbon atoms and 1 to 9 identical or different halogen atoms and

Ar represents phenyl, 2-pyridyl, 3-pyridyl or 4-pyridyl, in each case optionally monosubstituted or polysubstituted by identical or different substituents, the substituents chosen being: cyano, nitro, halogen, in each case straight-chain or branched alkyl, alkoxy or alkoxycarbonyl with 1 to 4 carbon atoms in the individual alkyl parts, in each case

straight-chain or branched halogenoalkyl or halogenoalkoxy with in each case 1 to 4 carbon atoms and 1 to 9 identical or different halogen atoms or a radical $-S(O)_n-R^6$,

wherein

$R^6$ represents amino, or represents in each case straight-chain or branched alkyl, alkylamino or dialkylamino with in each case 1 to 4 carbon atoms in the individual alkyl parts or halogenoalkyl with 1 to 4 carbon atoms and with1 to 9 identical or different halogen atoms and

n represents a number 0, 1 or 2.

2. 1-Aryl-5-alkoximinoalkylamino-pyrazoles of the formula (I) according to Claim 1, in which

R represents cyano or nitro, or represents a heterocyclic radical of the formula

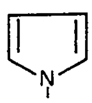 ; 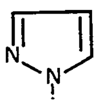 ; 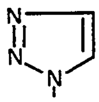 ; 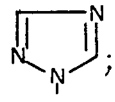 ; 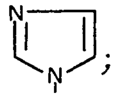 ; 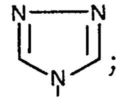 ; 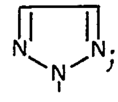 ; 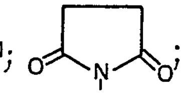 ; 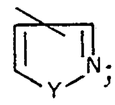 ;

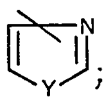 ; 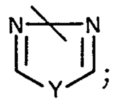 ; 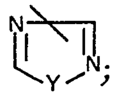 ; 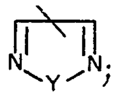 ; 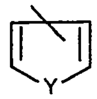 ; 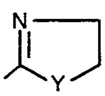 ; 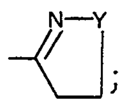 ; 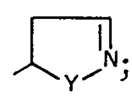 ; 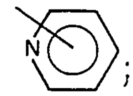 ;

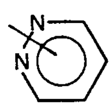 ; 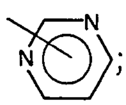 ; 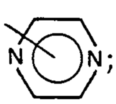 ; 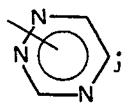 ; 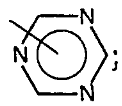 ; 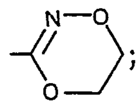 ; 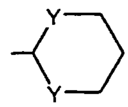 or 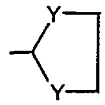

which is optionally mono-, di- or trisubstituted by identical or different substituents from the group comprising fluorine, chlorine, bromine, nitro, methyl, ethyl, n- and i-propyl, methoxy, ethoxy, n- and i-propoxy, methylthio, ethylthio, n- and i-propylthio and trifluoromethyl,

wherein

Y in each case represents oxygen, sulphur or an N-alkyl radical with 1 to 4 carbon atoms,

$R^1$ represents hydrogen, methyl, ethyl, n- or i-propyl or n-, i-,s- or t-butyl,

$R^2$ represents hydrogen, methyl, ethyl, n- or i-propyl or n-, i-, s- or t-butyl, or represents phenyl which is optionally mono-, di- or trisubstituted by identical or different substituents from the group comprising fluorine, chlorine, bromine, cyano, nitro, methyl, ethyl, methoxy, ethoxy, methylthio, ethylthio, methoxycarbonyl, ethoxycarbonyl, trifluoromethyl, trifluoromethoxy and trifluoromethylthio,

$R^3$ represents hydrogen, methyl, ethyl, n- or i-propyl, n-, i-, s- or t-butyl, allyl, 2-butenyl or 2-methyl-2-butenyl, or represents benzyl or phenyl, in each case mono-, di- or trisubstituted by identical or different substituents, the substituents chosen in each case being: fluorine, chlorine, bromine, cyano, nitro, methyl, ethyl, methoxy, ethoxy, methylthio, ethylthio or trifluoromethyl and

Ar represents phenyl, 2-pyridyl or 4-pyridyl, in each case optionally mono-, di-, tri-, tetra- or pentasubstituted by identical or different substituents, the substituents chosen being: cyano, nitro, fluorine, chlorine, bromine, iodine, methyl, ethyl, n- and i-propyl, n-, i-, s- and t-butyl, methoxy, ethoxy methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or a radical $-S(O)_n-R^6$,

wherein

$R^6$ represents amino, methyl, ethyl, methylamino, ethylamino, dimethylamino, diethylamino, fluorodichlormethyl, difluoromethyl, tetrafluoroethyl, trifluorochloroethyl or trifluoromethyl and

n represents a number 0, 1 or 2.

3. Process for the preparation of 1-aryl-5-alkoximinoalkylamino-pyrazoles of the formula (I)

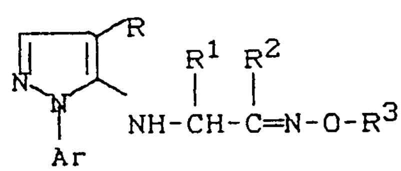 (I)

in which

R represents cyano or nitro, or represents a saturated or unsaturated, five-membered or six-membered heterocyclic radical which is optionally monosubstituted or polysubstituted by identical or different substituents, contains one to three identical or different hetero atoms from the group comprising nitrogen, oxygen and sulphur and can be linked via a carbon or a nitrogen atom, the substituents chosen being: halogen, nitro and in each case straight-chain or branched alkyl, alkoxy and alkylthio with in each case 1 to 4 carbon atoms or halogenoalkyl with 1 to

4 carbon atoms and 1 to 9 identical or different halogen atoms,

$R^1$ represents hydrogen, or represents straight-chain or branched alkyl with 1 to 8 carbon atoms,

$R_2$ represents hydrogen, or represents straight-chain or branched alkyl with 1 to 8 carbon atoms, or represents phenyl which is optionally monosubstituted or polysubstituted by identical or different substituents, the substituents chosen being: halogen, cyano, nitro, in each case straight-chain or branched alkyl, alkoxy, alkylthio or alkoxycarbonyl with in each case 1 to 4 carbon atoms in the individual alkyl parts or in each case straight-chain or branched halogenoalkyl, halogenoalkoxy and halogenoalkylthio with in each case 1 to 4 carbon atoms and 1 to 9 identical or different halogen atoms,

$R^3$ represents hydrogen, or represents straight-chain or branched alkyl with 1 to 8 carbon atoms, or represents straight-chain or branched alkenyl with 3 to 8 carbon atoms, or represents benzyl or phenyl, in each case optionally monosubstituted or polysubstituted by identical or different substituents from the group comprising halogen, cyano, nitro and in each case straight-chain or branched alkyl, alkoxy and alkylthio with in each case 1 to 4 carbon atoms and halogenoalkyl with 1 to 4 carbon atoms and 1 to 9 identical or different halogen atoms and

Ar represents phenyl, 2-pyridyl, 3-pyridyl or 4-pyridyl, in each case optionally monosubstituted or polysubstituted by identical or different substituents, the substituents chosen being: cyano, nitro, halogen, in each case straight-chain or branched alkyl, alkoxy or alkoxycarbonyl with 1 to 4 carbon atoms in the individual alkyl parts, in each case straight-chain or branched halogenoalkyl or halogenoalkoxy with in each case 1 to 4 carbon atoms and 1 to 9 identical or different halogen atoms or a radical $-S(O)_n-R^6$,

wherein

$R^6$ represents amino, or represents in each case straight-chain or branched alkyl, alkylamino or dialkylamino with in each case 1 to 4 carbon atoms in the individual alkyl parts or halogenoalkyl with 1 to 4 carbon atoms and with1 to 9 identical or different halogen atoms and

n represents a number 0, 1 or 2, characterized in that

(a) 5-amino-1-aryl-pyrazoles of the formula (II)

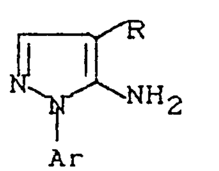

(II)

in which

R and Ar have the abovementioned meaning, are reacted with alkoximinoalkyl halides of the formula (III)

$$\text{Hal-CH}(R^1)\text{-C}(R^2)\text{=N-OR}^3 \quad \text{(III)}$$

in which

$R^1$, $R^2$ and $R^3$ have the abovementioned meaning and

Hal represents halogen,

if appropriate in the presence of a diluent and if appropriate in the presence of an acid-binding agent, or in that

(b) 5-dialkoxyalkylamino-pyrazoles of the formula (IV

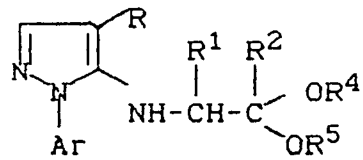

(IV)

in which

R, $R^1$, $R^2$ and Ar have die abovementioned meaning and

$R^4$ and $R^5$ in each case represent straight-chain or branched alkyl with 1 to 4 carbon atoms,

are reacted with hydroxylamine derivatives of the formula (V)

$$H_2N\text{-}O\text{-}R^3 \quad \text{(V)}$$

in which

$R^3$ has the abovementioned meaning, or acid addition salts thereof, if appropriate in the presence of a diluent and if appropriate in the presence of an acid-binding agent.

4. Herbicidal and plant growth-regulating agents, characterized in that they contain at least one 1-aryl-5-alkoximinoalkylamino-pyrazole of the formula (I) according to Claims 1 to 3.

5. Method of combating weeds, characterized in that 1-aryl-5-alkoximinoalkylamino-pyrazoles of the formula (I) according to Claims 1 to 3 are allowed to act on the weeds and/or environment.

6. Use of 1-aryl-5-alkoximinoalkylamino-pyrazoles of the formula (I) according to Claims 1 to 3 for combating weeds and/or as plant growth regulators.

7. Process for the preparation of herbicidal and/or plant growth-regulating agents, characterized in that 1-aryl-5-alkoximinoalkylamino-pyrazoles of the formula (I) according to Claims 1 to 3 are mixed with extenders and/or surface-active substances.

8. 5-Dialkoxyalkylamino-pyrazoles of the formula (IVa)

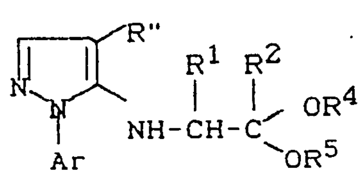

(IVa)

in which

R'' represents nitro, or represents a saturated or unsaturated, five-membered or six-membered heterocyclic radical which is optionally monosubstituted or polysubstituted by identical or different substituents, contains one to three identical or different hetero atoms from the group comprising nitrogen, oxygen and sulphur and can be linked via a carbon or

a nitrogen atom, the substituents chosen being: halogen, nitro and in each case straight-chain or branched alkyl, alkoxy and alkylthio with in each case 1 to 4 carbon atoms or halogenoalkyl with 1 to 4 carbon atoms and 1 to 9 identical or different halogen atoms,

$R^1$ represents hydrogen, or represents straight-chain or branched alkyl with 1 to 8 carbon atoms,

$R_2$ represents hydrogen, or represents straight-chain or branched alkyl with 1 to 8 carbon atoms, or represents phenyl which is optionally monosubstituted or polysubstituted by identical or different substituents, the substituents chosen being: halogen, cyano, nitro, in each case straight-chain or branched alkyl, alkoxy, alkylthio or alkoxycarbonyl with in each case 1 to 4 carbon atoms in the individual alkyl parts or in each case straight-chain or branched halogenoalkyl, halogenoalkoxy or halogenoalkylthio with in each case 1 to 4 carbon atoms and 1 to 9 identical or different halogen atoms,

$R^4$ represents straight-chain or branched alkyl with 1 to 4 carbon atoms,

$R^5$ represents straight-chain or branched alkyl with 1 to 4 carbon atoms and

Ar represents phenyl, 2-pyridyl, 3-pyridyl or 4-pyridyl, in each case optionally monosubstituted or polysubstituted by identical or different substituents, the substituents chosen being: cyano, nitro, halogen, in each case straight-chain or branched alkyl, alkoxy or alkoxycarbonyl with 1 to 4 carbon atoms in the individual alkyl parts, or straight-chain or branched halogenoalkyl or halogenoalkoxy with in each case 1 to 4 carbon atoms and 1 to 9 identical or different halogen atoms or a radical $-S(O)_n-R^6$, wherein

$R^6$ represents amino, or represents in each case straight-chain or branched alkyl, alkylamino, dialkylamino with in each case 1 to 4 carbon atoms in the individual alkyl parts or halogenoalkyl with 1 to 4 carbon atoms and with1 to 9 identical or different halogen atoms and

n represents a number 0, 1 or 2.

9. Process for the preparation of 5-dialkoxyalkylamino-pyrazoles of the formula (IVa)

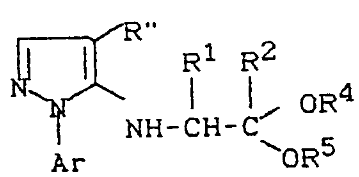

(IVa)

in which

R'' represents nitro, or represents a saturated or unsaturated, five-membered or six-membered heterocyclic radical which is optionally monosubstituted or polysubstituted by identical or different substituents, contains one to three identical or different hetero atoms from the group comprising nitrogen, oxygen and sulphur and can be linked via a carbon or a nitrogen atom, the substituents chosen being: halogen, nitro and in each case straight-chain or branched alkyl, alkoxy and alkylthio with in each case 1 to 4 carbon atoms or halogenoalkyl with 1 to 4 carbon atoms and 1 to 9 identical or different halogen atoms,

$R^1$ represents hydrogen, or represents straight-chain or branched alkyl with 1 to 8 carbon atoms,

$R_2$ represents hydrogen, or represents straight-chain or branched alkyl with 1 to 8 carbon atoms, or represents phenyl which is optionally monosubstituted or polysubstituted by identical or different substituents, the substituents chosen being: halogen, cyano, nitro, in each case straight-chain or branched alkyl, alkoxy, alkylthio or alkoxycarbonyl with in each case 1 to 4 carbon atoms in the individual alkyl parts or in each case straight-chain or branched halogenoalkyl, halogenoalkoxy or halogenoalkylthio with in each case 1 to 4 carbon atoms and 1 to 9 identical or different halogen atoms,

$R^4$ represents straight-chain or branched alkyl with 1 to 4 carbon atoms,

$R^5$ represents straight-chain or branched alkyl with 1 to 4 carbon atoms and

Ar represents phenyl, 2-pyridyl, 3-pyridyl or 4-pyridyl, in each case optionally monosubstituted or polysubstituted by identical or different substituents, the substituents chosen being: cyano, nitro, halogen, in each case straight-chain or branched alkyl, alkoxy or alkoxycarbonyl with 1 to 4 carbon atoms in the individual alkyl parts, or straight-chain or branched halogenoalkyl or halogenoalkoxy with in each case 1 to 4 carbon atoms and 1 to 9 identical or different halogen atoms or a radical $-S(O)_n-R^6$, wherein

$R^6$ represents amino, or represents in each case straight-chain or branched alkyl, alkylamino, dialkylamino with in each case 1 to 4 carbon atoms in the individual alkyl parts or halogenoalkyl with 1 to 4 carbon atoms and with1· to 9 identical or different halogen atoms and

n represents a number 0, 1 or 2,

characterized in that 5-amino-1-aryl-pyrazoles of the formula (IIa)

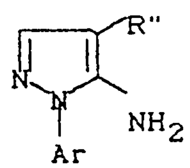

(IIa)

in which

R'' and Ar have the abovementioned meaning, are diazotized with nitrite compounds of the formula (XII)

$$R^7\text{-O-N=O} \quad \text{(XII)}$$

in which

$R^7$ represents hydrogen, an alkali metal cation or alkyl,

in the presence of a hydrogen halide acid or in the presence of a haloform and a catalyst acid, at temperatures between −20°C and +80°C, and the 5-halogenopyrazoles thus obtainable, of the formule (XIII)

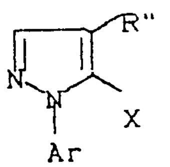

(XIII)

in which

R'' and Ar have the abovementioned meaning and

X represents halogen, in particular chlorine or bromine,

are reacted with dialkoxyalkylamines of the formula (XIV)

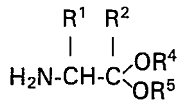

(XIV)

in which

$R^1$, $R^2$, $R^4$ and $R^5$ have the abovementioned meaning, at temperatures between +50°C and +250°C.

**Revendications**

1. 1-aryl-5-alcoximino-alkylamino-pyrazoles de formule (I):

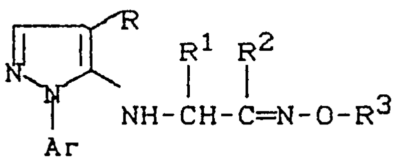

(I)

dans laquelle

R représente un groupe cyano, un groupe nitro ou un groupe hétérocyclique pentagonal ou hexagonal, saturé ou insaturé, éventuellement substitué une ou plusieurs fois de manière identique ou différente, contenant un à trois hétéroatomes identiques ou différents choisis parmi le groupe comprenant l'azote, l'oxygène ou le soufre, tout en pouvant être relié par un atome de carbone ou un atome d'azote, en choisissant, comme substituants: un atome d'halogène, un groupe nitro, ainsi qu'un groupe alkylthio, un groupe alcoxy et un groupe alkyle chacun à chaîne droite ou ramifiée et contenant chacun 1 à 4 atomes de carbone, ou un groupe halogénalkyle contenant 1 à 4 atomes de carbone et 1 à 9 atomes d'halogènes identiques ou différents,

$R^1$ représente un atome d'hydrogène ou un groupe alkyle à chaîne droite ou ramifiée contenant 1 à 8 atomes de carbone,

$R^2$ représente un atome d'hydrogène, un groupe alkyle à chaîne droite ou ramifiée contenant 1 à 8 atomes de carbone, ou un groupe phényle éventuellement substitué une ou plusieurs fois de manière identique ou différente, en choisissant, comme substituants: un atome d'halogène, un groupe cyano, un groupe nitro, un groupe alkyle, un groupe alcoxy, un groupe alkylthio ou un groupe alcoxycarbonyle chacun à chaîne droite ou ramifiée et contenant chacun 1 à 4 atomes de carbone dans les fractions alkyle individuelles ou un groupe halogénealkyle, un groupe halogénalkoxy ou un groupe halogénalkylthio chaque fois à chaîne droite ou ramifiée et contenant chacun 1 à 4 atomes de carbone et 1 à 9 atomes d'halogènes identiques ou différents,

$R^3$ représente un atome d'hydrogène, un groupe alkyle à chaîne droite ou ramifiée contenant 1 à 8 atomes de carbone, un groupe alcényle à chaîne droite ou ramifiée contenant 3 à 8 atomes de carbone, ou un groupe phényle ou un groupe benzyle éventuellement substitué chaque fois une ou plusieurs fois de manière identique ou différente par un atome d'halogène, un groupe cyano, un groupe nitro, un groupe alkyle, un groupe alcoxy ou un groupe alkylthio chaque fois à chaîne droite ou ramifiée et contenant chacun 1 à 4 atomes de carbone, ou par un groupe halogénalkyle contenant 1 à 4 atomes de carbone et 1 à 9 atomes d'halogènes identiques ou différents,

Ar représente un groupe phényle, un groupe 2-pyridyle, un groupe 3-pyridyle ou un groupe 4-pyridyle éventuellement substitué chaque fois une ou plusieurs fois de manière identique ou différente en choisissant, comme substituants: un groupe cyano, un groupe nitro, un atome d'halogène, un groupe alkyle, un groupe alcoxy ou un groupe alcoxycarbonyle chaque fois à chaîne droite ou ramifiée contenant 1 à 4 atomes de carbone dans les fractions alkyle individuelles, un groupe halogénalkyle ou un groupe halogénalcoxy chaque fois à chaîne droite ou ramifiée contenant chacun 1 à 4 atomes de carbone et 1 à 9 atomes d'halogènes identiques ou différents ou un radical $-S(O)_n-R^6$,

$R^6$ représentant un groupe amino, un groupe alkyle, un groupe alkylamino ou un groupe dialkylamino chaque fois à chaîne droite ou ramifiée et contenant chacun 1 à 4 atomes de carbone dans les fractions alkyle individuelles, ou encore un groupe halogénalkyle contenant 1 à 4 atomes de carbone et 1 à 9 atomes d'halogènes identiques ou différents, et

n représente un nombre de 0, 1 ou 2.

2. 1-aryl-5-alcoximino-alkylamino-pyrazoles de formule (I) selon la revendication 1, formule dans laquelle

R représente un groupe cyano, un groupe nitro ou un hétérocycle éventuellement substitué une à trois fois de manière identique ou différente par un atome de fluor, un atome de chlore, un atome de brome, un groupe nitro, un groupe méthyle, un groupe éthyle, un groupe n-propyle, un groupe i-propyle, un groupe méthoxy, un groupe éthoxy, un groupe n-propoxy, un groupe i-propoxy, un groupe méthylthio, un groupe éthylthio, un groupe n-propylthio, un groupe i-propylthio ou par un groupe trifluorométhyle, cet hétérocycle répondant aux formules:

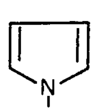 ; 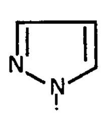 ; 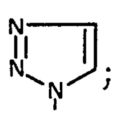 ; 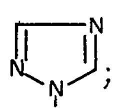 ; 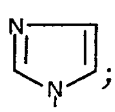 ; 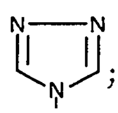 ; 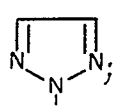 ; 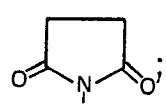 ; 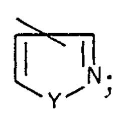 ;

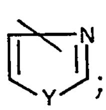; 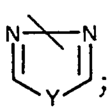; 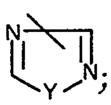; 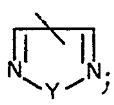; 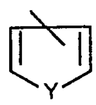; 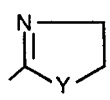; 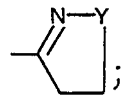; 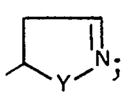; 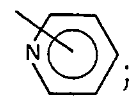;

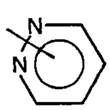; 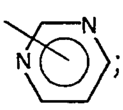; 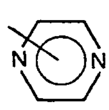; 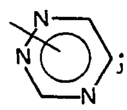; 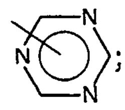; 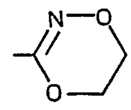; 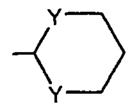 ou 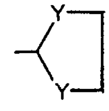

où

Y représente chaque fois un atome d'oxygène, un atome de soufre ou un groupe N-alkyle contenant 1 à 4 atomes de carbone,

$R^1$ représente un atome d'hydrogène, un groupe méthyle, un groupe éthyle, un groupe n-propyle, un groupe i-propyle, un groupe n-butyle, un groupe i-butyle, un groupe s-butyle ou un groupe t-butyle,

$R^2$ représente un atome d'hydrogène, un groupe méthyle, un groupe éthyle, un groupe n-propyle, un groupe i-propyle, un groupe n-butyle, un groupe i-butyle, un groupe s-butyle, un groupe t-butyle ou un groupe phényle éventuellement substitué une à trois fois de manière identique ou différente par un atome de fluor, un atome de chlore, un atome de brome, un groupe cyano, un groupe nitro, un groupe méthyle, un groupe éthyle, un groupe méthoxy, un groupe éthoxy, un groupe méthylthio, un groupe éthylthio, un groupe méthoxycarbonyle, un groupe éthoxycarbonyle, un groupe trifluorométhyle, un groupe trifluorométhoxy ou par un groupe trifluorométhyltio,

$R^3$ représente un atome d'hydrogène, un groupe méthyle, un groupe éthyle, un groupe n-propyle, un groupe i-propyle, un groupe n-butyle, un groupe i-butyle, un groupe s-butyle, un groupe t-butyle, un groupe allyle, un groupe 2-butényle, un groupe 2-méthyl-2-butényle ou un groupe benzyle ou phényle chaque fois substitué une à trois fois de manière identique ou différente en choisissant chaque fois, comme substituants: un atome de fluor, un atome de chlore, un atome de brome, un groupe cyano, un groupe nitro, un groupe méthyle, un groupe éthyle, un groupe méthoxy, un groupe éthoxy, un groupe méthylthio, un groupe éthylthio ou un groupe trifluorométhyle, et

Ar représente un groupe phényle, un groupe 2-pyridyle ou un groupe 4-pyridyle éventuellement substitué chaque fois une à cinq fois de manière identique ou différente en choisissant, comme substituants: un groupe cyano, un groupe nitro, un atome de fluor, un atome de chlore, un atome de brome, un atome d'iode, un groupe méthyle, un groupe éthyle, un groupe n-propyle, un groupe i-propyle, un groupe n-butyle, un groupe i-butyle, un groupe s-butyle, un groupe t-butyle, un groupe méthoxy, un groupe éthoxy, un groupe méthoxycarbonyle, un groupe éthoxycarbonyle, un groupe trifluorométhyle, un groupe trichlorométhyle, un groupe dichlorofluorométhyle, un groupe difluorochlorométhyle, un groupe chlorométhyle, un groupe dichlorométhyle, un groupe difluorométhyle, un groupe pentafluoréthyle, un groupe tétrafluoréthyle, un groupe trifluorochloréthyle, un groupe trifluoréthyle, un groupe difluorodichloréthyle, un groupe trifluorodichloréthyle, un groupe pentachloréthyle, un groupe trifluorométhoxy, un groupe trichlorométhoxy, un groupe dichlorofluorométhoxy, un groupe difluorochlorométhoxy, un groupe chlorométhoxy, un groupe dichlorométhoxy, un groupe difluorométhoxy, un groupe pentafluoréthoxy, un groupe tétrafluoréthoxy, un groupe trifluorochloréthoxy, un groupe trifluoréthoxy, un groupe difluorodichloréthoxy, un groupe trifluorodichloréthoxy, un groupe pentachloréthoxy ou un radical $-S(O)_n-R^6$,

$R^6$ représentant un groupe amino, un groupe méthyle, un groupe éthyle, un groupe méthylamino, un groupe éthylamino, un groupe diméthylamino, un groupe diéthylamino, un groupe fluorodichlorométhyle, un groupe difluorométhyle, un groupe tétrafluoroéthyle, un groupe trifluorochloréthyle ou un groupe trifluorométhyle, et

n représente un nombre de 0, 1 ou 2.

3. Procédé de préparation de 1-aryl-5-alcoximino-alkylamino-pyrazoles de formule (I):

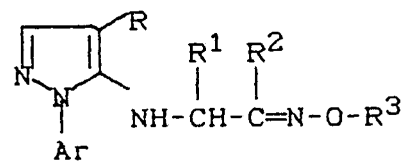 (I)

dans laquelle

R représente un groupe cyano, un groupe nitro ou un hétérocycle pentagonal ou hexagonal, saturé ou insaturé et éventuellement substitué une ou plusieurs fois de manière identique ou différente, contenant 1 à 3 hétéro-atomes identiques ou différents choisis parmi le groupe comprenant l'azote, l'oxygène et le soufre et pouvant être relié par un atome de carbone ou un atome d'azote, en choisissant, comme substituants: un atome d'halogène, un groupe nitro, ainsi qu'un groupe alkyle, un groupe alcoxy et un groupe alkylthio chaque fois à chaîne droite ou ramifiée et contenant chacun 1 à 4 atomes de carbone, ou un groupe halogénalkyle contenant 1 à 4 atomes de carbone et 1 à 9 atomes d'halogènes identiques ou différents,

$R^1$ représente un atome d'hydrogène ou un groupe alkyle à chaîne droite ou ramifiée contenant 1 à 8 atomes de carbone,

$R^2$ représente un atome d'hydrogène, un groupe alkyle à chaîne droite ou ramifiée contenant 1 à 8 atomes de carbone, ou un groupe phényle éventuellement substitué une ou plusieurs fois de manière identique ou différente, en choisissant, comme substituants: un atome d'halogène, un groupe cyano, un groupe nitro, un groupe alkyle, un groupe alcoxy, un groupe alkylthio ou un groupe alcoxycarbonyle chaque fois à chaîne droite ou ramifiée et contenant chacun 1 à 4 atomes de carbone dans les fractions alkyle individuelles ou un groupe halogénalkyle, un groupe halogénalcoxy ou un groupe halogénalkylthio chaque fois à chaîne droite ou ramifiée et contenant chacun 1 à 4 atomes de carbone et 1 à 9 atomes d'halogènes identiques ou différents,

$R^3$ représente un atome d'hydrogène, un groupe alkyle à chaîne droite ou ramifiée contenant 1 à 8 atomes de carbone, un groupe alcényle à chaîne droite ou ramifiée contenant 3 à 8 atomes de carbone, ou un groupe benzyle ou un groupe phényle éventuellement substitué chaque fois une ou plusieurs fois de manière identique ou différente par un atome d'halogène, un groupe cyano, un groupe nitro, un groupe alkyle, un groupe alcoxy ou un groupe alkylthio chaque fois à chaîne droite ou ramifiée et contenant chacun 1 à 4 atomes de carbone, ou un groupe halogénalkyle contenant 1 à 4 atomes de carbone et 1 à 9 atomes d'halogènes identiques ou différents, et

Ar représente un groupe phényle, un groupe 2-pyridyle, un groupe 3-pyridyle ou un groupe 4-pyridyle chaque fois éventuellement substitué une ou plusieurs fois de manière identique ou différente, en choisissant, comme substituants: un groupe cyano, un groupe nitro, un atome d'halogène, un groupe alkyle, un groupe alcoxy ou un groupe alcoxycarbonyle chaque fois à chaîne droite ou ramifiée contenant 1 à 4 atomes de carbone dans les fractions alkyle individuelles, un groupe halogénalkyle ou un groupe halogénalcoxy chaque fois à chaîne droite ou ramifiée contenant chacun 1 à 4 atomes de carbone et 1 à 9 atomes d'halogènes identiques ou différents ou un radical $-S(O)_n-R^6$,

$R^6$ représentant un groupe amino, un groupe alkyle, un groupe alkylamino ou un groupe dialkylamino chaque fois à chaîne droite ou ramifiée et contenant chacun 1 à 4 atomes de carbone dans les fractions alkyle individuelles, ou un groupe halogénalkyle contenant 1 à 4 atomes de carbone et 1 à 9 atomes d'halogènes identiques ou différents, et

n représente un nombre de 0, 1 ou 2,

caractérisé en ce qu'on fait reagier

(a) des 5-amino-1-aryl-pyrazoles de formule (II)

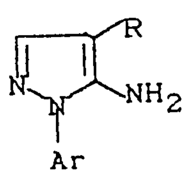

(II)

dans laquelle

R et Ar ont les significations indiquées ci-dessus, avec des halogénures d'alcoximino-alkyle de formule (III):

$R^1$ $R^2$
| |
$Hal-CH-C=N-OR^3$ (III)

dans laquelle

$R^1$, $R^2$ et $R^3$ ont les significations indiquées ci-desus, et

Hal représente un atome d'halogène,

éventuellement en présence d'un diluant et éventuellement en présence d'un agent fixateur d'acide, ou en ce que

(b) on fait réagir des 5-dialcoxy-alkylamino-pyrazoles de formule (IV):

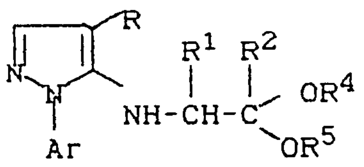

(IV)

dans laquelle

R, $R^1$, $R^2$ et Ar ont les significations indiquées ci-dessus,et

$R^4$ et $R^5$ représentent chacun un groupe alkyle à chaîne droite ou ramifiée contenant 1 à 4 atomes de carbone,

avec des dérivés d'hydroxylamine de formule (V):

$H_2N-O-R^3$ (V)

dans laquelle

$R^3$ a la signification indiquée ci-dessus, ou leurs sels d'addition d'acide, éventuellement en présence d'un diluant et éventuellement en présence d'un agent fixateur d'acide.

4. Herbicides et agents de régulation de la croissance des plantes, caractérisés en ce qu'ils contiennent au moins un 1-aryl-5-alcoximino-alkylamino--pyrazole de formule (I) selon les revendications 1 à 3.

5. Procédé en vue de combattre les plantes adventices, caractérisé en ce qu'on fait agir des 1-aryl-5-alcoximino-alkylamino-pyrazoles de formule (I) selon les revendications 1 à 3 sur les plantes adventices et/ou leur biotope.

6. Utilisation de 1-aryl-5-alcoximino-alkylamino--pyrazoles de formule (I) selon les revendications 1 à 3 en vue de combattre les plantes adventices et/ou comme régulateurs de la croissance des plantes.

7. Procédé de préparation d'agents herbicides et/ou de régulation de la croissance des plantes, caractérisé en ce qu'on mélange des 1-aryl-5-alcoximino-alkylamino-pyrazoles de formule (I) selon les revendications 1 à 3 avec des diluants et/ou des substances tensio-actives.

8. 5-dialcoxyalkylamino-pyrazoles de formule (IVa):

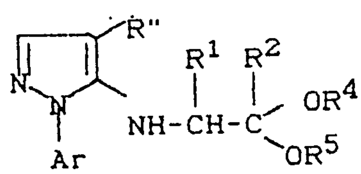

(IVa)

dans laquelle

R'' représente un groupe nitro ou un hétérocycle pentagonal ou hexagonal saturé ou insaturé éventuellement substitué une ou plusieurs fois de manière identique ou différente, contenant 1 à 3 hétéro-atomes identiques ou différents choisis parmi le groupe comprenant l'azote, l'oxygène et le soufre et pouvant être relié par un atome de carbone ou un atome

d'azote, en choisissant, comme substituants: un atome d'halogène, un groupe nitro, ainsi qu'un groupe alkyle, un groupe alcoxy ou un groupe alkylthio chaque fois à chaîne droite ou ramifiée et contenant chacun 1 à 4 atomes de carbone, ou un groupe halogénalkyle contenant 1 à 4 atomes de carbone et 1 à 9 atomes d'halogènes identiques ou différents,

$R^1$ représente un atome d'hydrogène ou un groupe alkyle à chaîne droite ou ramifiée contenant 1 à 8 atomes de carbone,

$R^2$ représente un atome d'hydrogène, un groupe alkyle à chaîne droite ou ramifiée contenant 1 à 8 atomes de carbone ou un groupe phényle éventuellement substitué une ou plusieurs fois de manière identique ou différente, en choisissant, comme substituants: un atome d'halogène, un groupe cyano, un groupe nitro, un groupe alkyle, un groupe alcoxy, un groupe alkylthio ou un groupe alcoxycarbonyle chaque fois à chaîne droite ou ramifiée et contenant chacun 1 à 4 atomes de carbone dans les fractions alkyle individuelles ou un groupe halogénalcoxy ou un groupe halogénalkylthio chaque fois à chaîne droite ou ramifiée et contenant chacun 1 à 4 atomes de carbone et 1 à 9 atomes d'halogènes identiques ou différents, et

$R^4$ représente un groupe alkyle à chaîne droite ou ramifiée contenant 1 à 4 atomes de carbone, et représente un groupe alkyle à chaîne droite ou ramifiée contenant 1 à 4 atomes de carbone, et

Ar représente un groupe phényle, un groupe 2-pyridyle, un groupe 3-pyridyle ou un groupe 4-pyridyle éventuellement substitué chacun une ou plusieurs fois de manière identique ou différente, en choisissant, comme substituants: un groupe cyano, un groupe nitro, un atome d'halogène, un groupe alkyle, un groupe alcoxy ou un groupe alcoxycarbonyle chaque fois à chaîne droite ou ramifiée et contenant 1 à 4 atomes de carbone dans les fractions alkyle individuelles, un groupe halogénalkyle ou un groupe halogénalcoxy chaque fois à chaîne droite ou ramifiée et contenant chacun 1 à 4 atomes de carbone et 1 à 9 atomes d'halogènes identiques ou différents, ou un radical $-S(O)_n-R^6$,

$R^6$ représentant un groupe amino, un groupe alkyle, un groupe alkylamino ou un groupe dialkylamino chaque fois à chaîne droite ou ramifiée et contenant chacun 1 à 4 atomes de carbone dans les fractions alkyle individuelles, ou un groupe halogénalkyle contenant 1 à 4 atomes de carbone et 1 à 9 atomes d'halogènes identiques ou différents, et

n représente un nombre de 0, 1 ou 2.

9. Procédé de préparation de 5-dialcoxy-alkylamino-pyrazoles de formule (IVa):

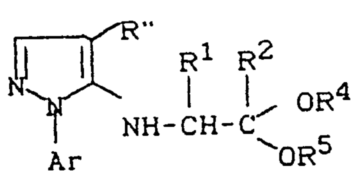

(IVa)

dans laquelle

R'' représente un groupe nitro ou un hétérocycle pentagonal ou hexagonal saturé ou insaturé, éventuellement substitué une ou plusieurs fois de manière identique ou différente, contenant un à trois hétéroatomes identiques ou différents choisis parmi le groupe comprenant l'azote, l'oxygène et le soufre et pouvant être relié par un atome de carbone ou un atome d'azote, en choisissant comme substituants: un atome d'halogène, un groupe nitro, ainsi qu'un groupe alkyle, un groupe alcoxy ou un groupe alkylthio chaque fois à chaîne droite ou ramifiée et contenant chacun 1 à 4 atomes de carbone, ou un groupe halogénalkyle contenant 1 à 4 atomes de carbone et 1 à 9 atomes d'halogènes identiques ou différents,

$R^1$ représente un atome d'hydrogène ou un groupe alkyle à chaîne droite ou ramifiée contenant 1 à 8 atomes de carbone,

$R^2$ représente un atome d'hydrogène, un groupe alkyle à chaîne droite ou ramifiée contenant 1 à 8 atomes de carbone ou un groupe phényle éventuellement substitué une ou plusieurs fois de manière identique ou différente, en choisissant, comme substituants: un atome d'halogène, un groupe cyano, un groupe nitro, un groupe alkyle, un groupe alcoxy, un groupe alkylthio ou un groupe alcoxycarbonyle chaque fois à chaîne droite ou ramifiée et contenant chacun 1 à 4 atomes de carbone dans les fractions alkyle individuelles, ou un groupe halogénalkyle, un groupe halogénalcoxy ou un groupe halogénalkylthio chaque fois à chaîne droite ou ramifiée et contenant chacun 1 à 4 atomes de carbone et 1 à 9 atomes d'halogènes identiques ou différents,

$R^4$ représente un groupe à chaîne droite ou ramifiée contenant 1 à 4 atomes de carbone,

$R^5$ représente un groupe alkyle à chaîne droite ou ramifiée contenant 1 à 4 atomes de carbone, et

Ar représente un groupe phényle, un groupe 2-pyridyle, un groupe 3-pyridyle ou un groupe 4-pyridyle chaque fois éventuellement substitué une ou plusieurs fois de manière identique ou différente, en choisissant, comme substituants: un groupe cyano, un groupe nitro, un atome d'halogène, un groupe alkyle, un groupe alcoxy ou un groupe alcoxycarbonyle chaque fois à chaîne droite ou ramifiée et contenant chacun 1 à 4 atomes de carbone dans les fractions alkyle individuelles, un groupe halogénalkyle ou un groupe halogénalcoxy chaque fois à chaîne droite ou ramifiée et contenant chacun 1 à 4 atomes de carbone et 1 à 9 atomes d'halogènes identiques ou différents, ou un radical $-S(O)_n-R^6$,

$R^6$ représentant un groupe amino, un groupe alkyle, un groupe alkylamino ou un groupe dialkylamino chaque fois à chaîne droite ou ramifiée contenant chacun 1 à 4 atomes de carbone dans les fractions alkyle individuelles, ou un groupe halogénalkyle contenant 1 à 4 atomes de carbone et 1 à 9 atomes d'halogènes identiques ou différents, et

n représente un nombre de 0, 1 ou 2, caractérisé en ce qu'on effectue la diazotation de 5-amino-1-aryl-pyrazoles de formule (IIa):

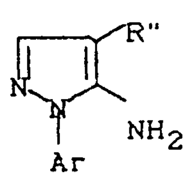

(IIa)

dans laquelle

R'' et Ar ont les significations indiquées ci-dessus, avec des composés nitrites de formule (XII):

$R^7$-O-N=O (XII)

dans laquelle

$R^7$ représente un atome d'hydrogène, un cation d'un métal alcalin ou un groupe alkyle,

en présence d'un hydracide halogéné ou en présence d'un haloforme et d'un acide catalytique, à des températures comprises entre −20°C et +80°C et on fait réagir les 5-halogénopyrazoles ainsi obtenues de formule (XIII):

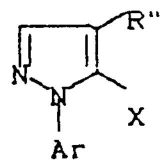

(XIII)

dans laquelle

R'' et Ar ont les significations indiquées ci-dessus, et

X représente un atome d'halogène, en particulier, un atome de chlore ou un atome de brome, avec des dialcoxyalkylamines de formules (XIV):

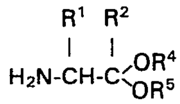

(XIV)

dans laquelle

$R^1$, $R^2$, $R^4$ et $R^5$ ont les significations indiquées ci-dessus,

à des températures comprises entre + 50°C et + 250°C.